# EUROPEAN PATENT APPLICATION

(11) **EP 3 851 192 A1**
(43) Date of publication of application: **21.07.2021**
(21) Application number: 21151213.2
(22) Date of filing: 12.01.2021
(51) Int. Cl.: B01L 3/02, C12M 1/26, B29C 64/106, B29C 64/295, G01N 35/10, B01L 7/00

(54) **DISPENSING SYSTEM WITH TEMPERATURE-REGULATION**

(30) Priority: 17.01.2020 SE 2050038
(71) Applicant: Cellink AB, 41346 Gothenburg (SE)
(72) Inventor: PERSSON, Sebastian, 412 58 Göteborg (SE); ANDRÉN, Anton, 431 38 Mölndal (SE); MARTINEZ, Hector, 413 09 Göterborg (SE); GATENHOLM, Erik, 411 33 Göteborg (SE)
(74) Representative: Zacco Sweden AB

(57) **Abstract**

A dispensing system (1) comprising a material cartridge (2) provided with a nozzle (3), a temperature-regulating unit (4) provided with a nozzle temperature-regulating zone (5) arranged to regulate the temperature of the material cartridge nozzle (3) when the material cartridge nozzle (3) is arranged within the nozzle temperature-regulating zone (5), and a positioning unit (6) arranged for movement of the material cartridge (2) and the nozzle temperature-regulating zone (5) relative to each other between a first position (A) and a second position (B). In the first position (A) the material cartridge nozzle (3) is arranged within the nozzle temperature-regulating zone (5), and in the second position (B) at least a leading end portion (3a) of the material cartridge nozzle (3) is arranged outside the nozzle temperature-regulating zone (5).

## Description

### TECHNICAL FIELD

The present disclosure relates to the field of dispensing systems with temperature regulation and to multi-dispensing systems, 3D bioprinters and biodispensing systems comprising one or more such dispensing system.

### BACKGROUND ART

A fluid dispensing system is a device, machine or equipment that is responsible for dispensing a fluid in controlled quantities and apply it on a desired area. Being able to precisely dispense fluids onto a specific point in a controlled way is a main characteristic of fluid dispensing systems, and may be utilized in 3D printing

Dispensing systems can use either air pressure or positive displacement to dispense fluids in a controlled way. Dispensing systems can be manually or automatically operated. They can be used in small volume and mass production applications and in various applications (e.g., electronics industry, automotive industry, life science industry) that demand accurate, uniform, process-controlled, and high throughput of repeatable depositions.

Dispensing systems may be used in life science applications such as liquid handling/dispensing of low (pL to nL range) and medium (microliter range) volumes of cell culture reagents, compound dosing, combinatorial dispensing, titration, dispensing RNA samples for PCR analysis and in 3D bioprinting.

Examples of fluids used in dispensing systems within the life science industry include cell culture reagents such as cell culture media, growth factors, cell culture ingredients, animal-derived supplements, non-animal origin supplements and hydrogels for 3D cell culture.

Heated dispensing heads are often used in 3D-printing, as well as in precision dispensing systems, because the heating function can allow the printability/dispensing of materials by increasing the temperature of the material above or close to its melting or gelation point to make it flow through a needle, nozzle or orifice to form a droplet and/or filament without clogging the nozzle.

Non-heated dispensing heads are used in dispensing systems and 3D bioprinters to dispense polysaccharide hydrogels such as alginate, cellulose, xanthan gum, gellan gum, typically at a concentration below 1% w/w, as well as non-viscous cell culture reagents, such as cell culture media with and without growth factors and supplements. The above liquids and hydrogels can be processed and dispensed at room temperature (20°C to 27°C) for short (within minutes) and long (hours) periods of time without the fluid undergoing material decomposition, polymerization and/or crosslinking that may affect the dispensing accuracy and precision of the fluid/material/hydrogel.

Many common hydrogels used for 2D and 3D cell culture, e.g. Matrigel®, require a cool temperature below 4°C to be able to be processed over short (within minutes) and long (hours) periods of time without inducing polymerization in the dispensing nozzle.

The ability to keep the temperature of the dispensing head and any fluid contained therein stable and accurate over long periods of time (hours) is of utmost importance. If the printhead is idle, i.e. there is no extrusion through the nozzle, temperature sensitive fluid left in the nozzle may turn stiff in a couple of seconds and cause clogging in the nozzle. Precise control of temperature and temperature range for dispensing materials at a high accuracy and precision for long periods of time provides additional challenges that remain to be addressed.

### SUMMARY OF THE INVENTION

It is an object of the present disclosure to provide a dispensing system having a temperature-regulating unit arranged to regulate the temperature of a material cartridge nozzle. Further objects are to provide a multi-dispensing system, a 3D bioprinter and a biodispensing system comprising one or more dispensing systems, and a method of regulating the temperature of a nozzle of a material cartridge. Yet a further object is to provide a computer program comprising computer program code which, when executed, regulates the temperature of the material cartridge nozzle.

The invention is defined by the appended independent patent claims. Nonlimiting embodiments emerge from the dependent claims, the appended drawings and the following description.

According to a first aspect there is provided a dispensing system, which comprises a material cartridge provided with a nozzle, a temperature-regulating unit provided with a nozzle temperature-regulating zone arranged to regulate the temperature of the material cartridge nozzle when the material cartridge nozzle is arranged within the nozzle temperature-regulating zone, and a positioning unit arranged for movement of the material cartridge and the nozzle temperature-regulating zone relative to each other between a first position and a second position. In the first position the material cartridge nozzle is arranged within the nozzle temperature-regulating zone, and in the second position at least a leading end portion of the material cartridge nozzle is arranged outside the nozzle temperature-regulating zone.

The dispensing system may be used in a 3D printer, which may be a 3D bioprinter. A 3D bioprinter utilizes 3D printing and 3D printing-like techniques to combine cells, growth factors, and biomaterials to fabricate tissue-like or tissue analogue structures that imitate natural tissue characteristics. Generally, 3D bioprinting utilizes the layer-by-layer method to deposit/dispense dispensing contents, such as materials known as bioinks or hydrogel to create tissue-like structures that are later used in life science and tissue engineering fields. Bioprinting covers a broad range of biomaterials or bioinks.

The dispensing system may be used as or in a biodispensing system. A biodispensing system is a system that is capable of precisely dispensing material onto a specific point in a controlled way.

Material dispensed by the dispensing system and filled in the material cartridge may be temperature-sensitive materials. The material may be a material that require a temperature above room temperature to be processed, i.e. by increasing the temperature of the material above or close to its melting or gelation point the material can flow through the nozzle without clogging the nozzle. Such materials may be gelatine-based materials such as e.g. GelMA from Cellink®. The material may alternatively be a material requiring a cool temperature, such as for example below 4°C, to be able to be processed without inducing polymerization and clogging in the dispensing nozzle. ECM hydrogels, which are extracellular matrix-derived solutions such as gelatinous protein mixtures, extracellular matrix proteins in solution (in acidic, neutral or basic pH), and basement membrane matrices such as Matrigel®, Geltrex® and Cultrex® Basement Membrane Extract, are all temperature-sensitive materials that require a low temperature for dispensing.

When the material cartridge nozzle is arranged within the nozzle temperature-regulating zone there may be no dispensing of material through the nozzle. When the material cartridge and the nozzle-temperature regulating zone are in the second position relative to each other a dispensing action may be allowed. Before and after a dispensing action the material cartridge nozzle may be arranged within the nozzle-temperature regulating zone.

In a dispensing system without a nozzle-temperature regulating zone, temperature-sensitive material left in the material cartridge nozzle after a dispensing action may turn stiff in a couple of seconds and cause clogging in the nozzle. By regulating the temperature of the nozzle by means of the nozzle temperature-regulating zone before, after and between dispensing actions, the clogging problem may be reduced.

The temperature-regulating unit may be arranged to regulate the temperature of the material cartridge nozzle to a temperature within a range of -10°C to 20°C.

The temperature may be regulated to a temperature within a range of -10°C to 15°C, -10°C to 10°C, -10°C to 5°C, -10°C to 0°C, -10°C to -5°C, -5°C to 20°C, -5°C to 15°C, -5°C to 10°C, -5°C to 5°C, -5°C to 0°C, 0°C to 20°C, 0°C to 15°C, 0°C to 10°C, 0°C to 5°C, 5°C to 20°C, 5°C to 15°C or 5°C to 10°C, 10°C to 20°C, 10°C to 15°C or 15°C to 20°C.

By cooling the nozzle before, after and between dispensing actions, the clogging problem may be reduced when dispensing materials which require a cool temperature to be able to be processed.

The temperature-regulating unit may be alternatively or additionally be arranged to regulate the temperature of the material cartridge nozzle to a temperature within a range of 20 to 120°C.

The temperature may be regulated to a temperature within a range of 20°C to 100°C, 20°C to 80°C, 20°C to 60°C, 20°C to 40°C, 40°C to 120°C, 40°C to 100°C, 40°C to 80°C, 40°C to 60°C, 60°C to 120°C, 60°C to 100°C, 60°C to 80°C, 80°C to 120°C, 80°C to 100°C or 100°C to 120°C.

By heating the nozzle before, after and between dispensing actions, the clogging problem may be reduced when dispensing materials that require a temperature above room temperature to be able to be processed.

The temperature-regulating unit may comprise a hollow compartment, at least a portion thereof forming the temperature regulating zone. The hollow compartment may comprise a nozzle opening, wherein when the material cartridge and the nozzle temperature-regulating zone are in the first position relative to each other the material cartridge nozzle is arranged in the hollow compartment in the temperature regulating zone, and when the material cartridge and the nozzle temperature-regulating zone are in the second position relative to each other at least the leading end portion of the material cartridge nozzle extends out of the hollow compartment through the nozzle opening.

The nozzle opening may be a vertical nozzle opening, a horizontal nozzle opening, a nozzle opening having a direction of extension in any direction between a substantially vertical and a substantially horizontal nozzle opening, or the nozzle opening may be a pivotable nozzle opening.

The temperature-regulating unit may further comprise a Peltier element arranged to transfer heat from the compartment to a heat-transferring element connected to the Peltier element and arranged to transfer heat generated by the Peltier element and dissipate the transferred heat away from the Peltier element.

The heat-transferring element may be a fan.

The heat-transferring element may be a liquid cooling unit comprising a liquid coolant.

In an alternative embodiment the temperature-regulating unit may further comprise a Peltier element arranged to heat the compartment.

When the material cartridge and the nozzle temperature-regulating zone are in the second position relative to each other the material cartridge may be arranged in a dispensing position.

When the material cartridge and the temperature-regulating zone are in the first position relative to each other the material cartridge may be arranged in a non-dispensing position.

The relative movement of the material cartridge and the temperature-regulating zone between the first and second position may be a substantially vertical movement.

The material cartridge may be stationary and movement of the material cartridge and the nozzle temperature-regulating zone relative to each other between the first position and the second position may be accomplished by movement of the nozzle temperature-regulating zone.

The nozzle temperature-regulating zone may be amoveable portion of a stationary hollow compartment.

The nozzle temperature-regulating zone may be moveable in a vertical direction, in a horizontal direction, in any direction between a substantially vertical and a substantially horizontal direction.

The nozzle temperature-regulating zone may be stationary and movement of the material cartridge and the nozzle temperature-regulating zone relative to each other between the first position and the second position may be accomplished by movement of the material cartridge.

The movement of the material cartridge and the nozzle temperature-regulating zone relative to each other between the first position and the second position may be accomplished by movement of both the material cartridge and the nozzle temperature-regulating zone.

The material cartridge may comprise a body and a plunger arranged to be linearly pulled and pushed along the inside of the body, wherein upon movement of the material cartridge the position of the plunger relative the body is kept constant.

The dispensing system may further comprise a dispensing regulating unit arranged to regulate a dispensing action of the material cartridge.

The material cartridge and the dispensing regulating unit may be fixed relative to each other upon movement of the material cartridge and the nozzle temperature-regulating zone relative to each other between the first position and the second position.

The positioning unit may comprise a rack and gear which upon interaction moves the material cartridge and the nozzle temperature-regulating zone relative to each other between the first position and the second position.

According to a second aspect there is provided a multi-dispensing system for a 3D printer comprising two or more of the dispensing systems described above.

According to a third aspect there is provided a 3D bioprinter or biodispensing system comprising one or more of the dispensing systems described above or one or more multi-dispensing systems described above.

According to a fourth aspect there is provided a method of regulating the temperature of a nozzle of a material cartridge, the method comprising providing a material cartridge with a nozzle, providing a temperature-regulating unit provided with a nozzle temperature-regulating zone, and providing a positioning unit for movement of the material cartridge and the nozzle temperature-regulating zone relative to each other between a first position and a second position. In the first position the temperature-regulating unit is arranged to regulate the temperature of the material cartridge nozzle, and in the second position at least a leading end portion of the material cartridge nozzle is arranged outside the nozzle temperature-regulating zone.

A computer program comprising computer program code may be used which, when executed, causes a positioning unit to move a material cartridge and a nozzle temperature-regulating zone relative to each other between a first position and a second position, wherein in the first position the temperature-regulating unit may be arranged to regulate the temperature of the material cartridge nozzle, and in the second position at least a leading end portion of the material cartridge nozzle may be arranged outside the nozzle temperature-regulating zone.

### BRIEF DESCRIPTION OF THE DRAWINGS

Fig. 1a shows a dispensing system with a temperature-regulating unit in a dispensing position and Fig. 1b shows a dispensing system with a temperature-regulating unit in an idle/non-dispensing position.
Fig. 2a shows a dispensing system with a temperature-regulating unit in a dispensing position, Fig. 2b and Fig. 2c show different embodiments of a dispensing system with a temperature-regulating unit in an idle/non-dispensing position.
Fig. 3a shows a dispensing system with a temperature-regulating unit in a dispensing position, and Fig. 3b shows the dispensing system with the temperature-regulating unit in an idle/non-dispensing position.
Fig. 4a shows a dispensing system with removed material cartridge. In Fig. 4b the material cartridge is put into the dispensing system in the idle/non-dispensing position and in Fig. 4c it is illustrated how the material cartridge is locked in place in the dispensing system.
Fig. 5b shows a multi-dispensing system illustrating that the system can be made smaller and more compact if the material cartridge nozzle is moved into the temperature-regulating zone when in a non-printing mode as compared to the multi-dispensing system in Fig. 5a where there is no movement of the material cartridge nozzle into the temperature-regulating zone when in a non-printing mode.
Fig. 6 illustrates a dispensing system comprising a temperature-regulating unit comprising a Peltier element.

### DETAILED DESCRIPTION

Many common hydrogels require a cool temperature to be able to be processed through pipetting or 3D printing without inducing polymerization in the dispensing nozzle, which in turn affects the dispensing accuracy and precision. Other materials require a temperature above room temperature to be dispensable/printable. Heating can allow the printability/dispensing of the material by increasing the temperature of the material above or close to its melting or gelation point to make it flow through a nozzle to form a droplet and/or filament. It is desirable to keep the temperature of the dispensing material stable and accurate over short (seconds) and long periods of time (hours) before, between and after a dispensing action. If there is no extrusion through a dispensing nozzle, temperature sensitive material left in the nozzle may turn stiff in a couple of seconds and cause clogging in the nozzle.

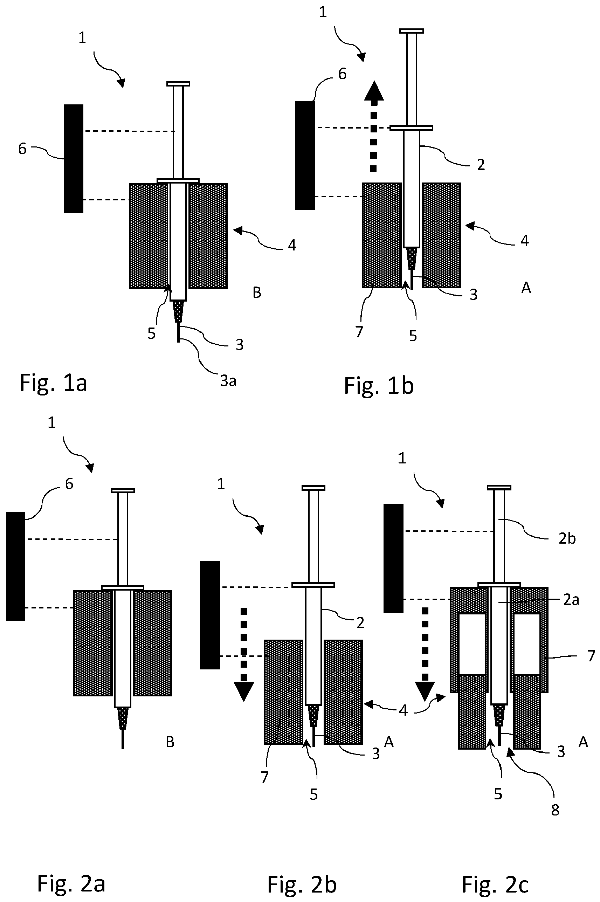

In Figs 1a, 1b, 2a-2c, 3a, 3b, 4a-4c is shown a dispensing system 1 for a 3D printer, which printer may be a bioprinter. The dispensing system 1 comprises a material cartridge 2 provided with a nozzle 3. The material cartridge 2 may, as shown in the figures, comprise a body 2a and a plunger or piston 2b that fits tightly within the body. The material cartridge may be a cylindrical tube, a barrel etc. The plunger may be arranged to be linearly pulled and pushed along the inside of the body. The material cartridge 2 may be a syringe or a modified syringe.

The dispensing system 1 is provided with a temperature-regulating unit 4 provided with a nozzle temperature-regulating zone 5, which is arranged to regulate the temperature of the material cartridge nozzle 3 when the material cartridge nozzle 3 is arranged within the nozzle temperature-regulating zone 5, see Figs 1b, 2b and 2c. The temperature-regulating unit 4 regulates the temperature of the nozzle 3 and any content contained therein. The nozzle temperature-regulating zone 5, may regulate the temperature of the nozzle 3 only. Alternatively, it may also regulate the temperature of the material cartridge 2 or at least a portion of the material cartridge 2 body (and any content therein) to which the nozzle 3 is attached.

The temperature-regulating unit 4 may be arranged to regulate the temperature of the material cartridge nozzle 3 via the nozzle temperature-regulating zone 5 to a temperature within a range of -10 to 20°C. Thereby, materials requiring a cool temperature, such as below 4°C, may be processed by keeping the temperature of the dispensing material in the nozzle 3 (and in the material cartridge 2) stable and accurate before, between and after a dispensing action. Any dispensing material left in the nozzle 3 after a dispensing action will be cooled by the nozzle temperature-regulating zone 5 to prevent clogging of the material in the nozzle 3.

In an alternative or additional embodiment, the temperature-regulating unit 4 may be arranged to regulate the temperature of the material cartridge nozzle 3 via the temperature-regulating zone 5 to a temperature within a range of 20 to 120°C. Thereby, materials requiring a temperature above room temperature may be processed by keeping the temperature of the dispensing material in the nozzle 3 (and in the material cartridge 2) stable and accurate before, between and after a dispensing action. Any dispensing material left in the nozzle 3 after a dispensing action will be heated by the nozzle temperature-regulating zone 5 to prevent clogging of the material in the nozzle 3.

Hence, in one embodiment the same temperature-regulating unit 4 may be arranged to regulate the temperature of the material cartridge nozzle 3 via the temperature-regulating zone 5 to any temperature within a range of -10°C to 120°C.

The dispensing system further comprises a positioning unit 6 arranged for movement of the material cartridge 2 and the nozzle temperature-regulating zone 5 relative to each other between a first position A and a second position B. In the first position A the material cartridge nozzle 3 is arranged within the nozzle temperature-regulating zone 5. In the second position B at least a leading end portion 3a of the material cartridge nozzle 3 is arranged outside the nozzle temperature-regulating zone 5, see e.g. Figs 1a and 2a. This means that only a portion of the nozzle 3, a major part of the nozzle 3 or the whole nozzle 3 is arranged outside the nozzle temperature-regulating zone 5 in the second position B. Outside the nozzle temperature-regulating zone 5 there is no temperature-regulation of the nozzle 3.

When the material cartridge 2 and the nozzle temperature-regulating zone 5 are in the second position B relative to each other (Figs 1a, 2a) the material cartridge 2 may be arranged in a dispensing position. In the dispensing position a portion of a material filled in the material cartridge 2 may be arranged to be supplied on a substrate through the nozzle 3. The dispensing may be an air powered dispensing using air pressure that is outputted by an air compressor or a similar device and push a material in the material cartridge on a piston or piston-like component that in turn push a material in a barrel out of the nozzle. The dispensing may be a positive displacement dispensing using compressed air to push a piston inside a barrel by means of a mechanical force that can be generated by electric stepper motors. They are ideal for instance for materials that change viscosity over time generally and for precise control of flow rate and volume of the dispensed material.

When the material cartridge 2 and the temperature-regulating zone 5 are in the first position A relative to each other (Figs. 1b, 2b, 2c) the material cartridge 2 may be arranged in a non-dispensing position. Such non-dispensing position may be a position taken prior to and after a dispensing action.

As seen in the figures, the relative movement of the material cartridge 2 and the temperature-regulating zone 5 between the first A and second position B may be a substantially vertical movement. Alternatively, the relative movement may be a substantially horizontal movement or any a relative movement between a substantially vertical and a substantially horizontal movement.

In the embodiment shown in Figs 2b and 2c the material cartridge 2 is stationary, i.e. fixed, and movement of the material cartridge 2 and the nozzle temperature-regulating zone 5 relative to each other between the first position A and the second position B may be accomplished by movement of the nozzle temperature-regulating zone 5.

In the embodiment shown in Fig. 1b, the nozzle temperature-regulating zone 5 is stationary, i.e. fixed, and movement of the material cartridge 2 and the nozzle temperature-regulating zone 5 relative to each other between the first position A and the second position B may be accomplished by movement of the material cartridge 2.

In a non-shown embodiment, movement of the material cartridge 2 and the nozzle temperature-regulating zone relative to each other may be accomplished by movement of both the material cartridge 2 and the nozzle temperature-regulating zone 5.

As shown in the figures, the temperature-regulating unit 4 may comprise a hollow compartment 7, wherein at least a portion thereof forms the temperature-regulating zone 5. The compartment 7 may be a hollow cylinder, cube or cuboid. The compartment may be in one piece. Alternatively, the compartment may comprise two or more pieces joined together to form the hollow compartment. The compartment may be configured such that also at least a portion of the material cartridge 2 is containable in the hollow compartment 7. The hollow compartment 7 may be a circumferential chamber, or an at least partially circumferential chamber, configured to surround at least a portion of the nozzle 3 (and the material cartridge 2) when the nozzle is within the temperature-regulating zone 5. The inner diameter of the compartment 7 may correspond to the outer diameter of the material cartridge 2. The hollow compartment 7 may comprise a nozzle opening 8, wherein when the material cartridge 2 and the nozzle temperature-regulating zone 5 are in the first position A relative to each other the material cartridge nozzle 3 is arranged in the hollow compartment 7 in the temperature regulating zone 5 as shown in Figs 1b, 2b, 2c. The compartment 7 may comprise an opening opposite to the nozzle opening 8 through which a portion of the material cartridge opposite to the nozzle may extend. When the material cartridge 2 and the nozzle temperature-regulating zone 5 are in the second position B relative to each other at least the leading end portion 3a of the material cartridge nozzle 3 may extend out of the hollow compartment 7 through the nozzle opening 8 as shown in Figs 1a and 2a. The nozzle opening may be a vertical nozzle opening, a horizontal nozzle opening or a pivotable nozzle opening. The compartment may be made of aluminium. The compartment 7 or at least the temperature-regulating zone 5 thereof may be a heat-exchanger. It may be provided with fluid channels for heated fluid (gas or liquid)/cooled fluid or electrical heating/cooling means within the compartment wall, at an inside of the compartment wall facing the nozzle, at an outer surface of the compartment, such as to heat or cool the compartment 7 or at least the temperature-regulating zone to a pre-set temperature. Thereby, regulating the temperature of the nozzle 3 (and at least a portion of the material cartridge 2).

The nozzle temperature-regulating zone 5 may be, as shown in Fig. 2c, a moveable portion of a stationary hollow compartment 7. The moveable portion may be a portion extending out of the compartment 7 when the material cartridge 2 and the nozzle temperature-regulating zone 5 are in the first position A relative to each other, such that the nozzle 3 is arranged in the nozzle temperature-regulating zone. When in the second position B relative to each other, the moveable portion is retracted into the hollow compartment such that at least the nozzle extends out of the compartment. The nozzle temperature-regulating zone 5 may be moveable in a vertical direction, in a horizontal direction, in any direction between a substantially vertical and a substantially horizontal direction.

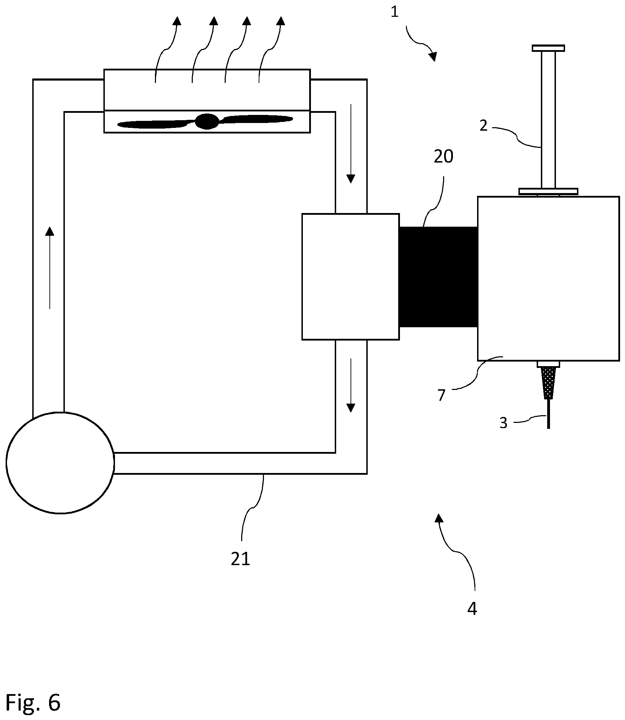

In Fig. 6 the temperature regulating unit 4 further comprises a Peltier element 20 arranged to transfer heat from the compartment 7 to a heat transferring element 21 connected to the Peltier element 20 and arranged to transfer heat generated by the Peltier element 20 and dissipate the transferred heat away from the Peltier element 20. The compartment 7 may be isolated such as not transferring any heat to the surroundings. Heat is only transferred from the compartment at the side thereof facing/being in contact with the Peltier element. The Peltier element 20 may be in physical contact with an outer wall of the compartment 7. The heat transferring element 21 may be a fan (not illustrated). As shown in Fig. 6, the heat transferring element 21 may be a liquid cooling unit 21 comprising a liquid coolant.

In the alternative embodiment when the nozzle is not cooled but heated by the temperature-regulating unit 4, the temperature regulating unit 4 may further comprise a Peltier element 20 arranged to heat the compartment 7 (not shown).

When the material cartridge 2 comprises a body 2a and a plunger 2b, movement of the material cartridge 2 relative the nozzle-temperature regulating zone 5 may be accomplished such that the position of the plunger 2b relative the body 2a is kept constant, as is illustrated in the figures. Thereby, when lifting the material cartridge, both the plunger, the body and their relative positions are controlled.

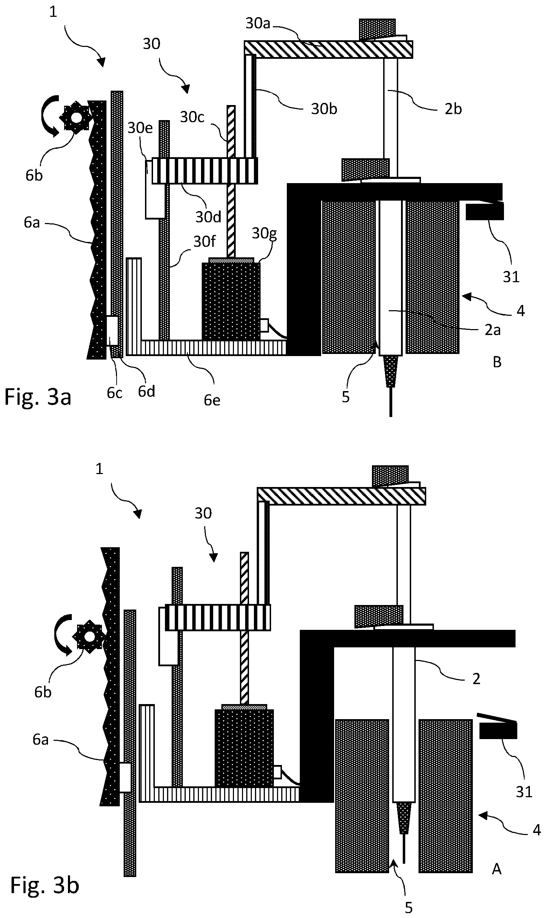

As shown in *e.g.* Fig. 3a, the dispensing system 1 may comprise a dispensing regulating unit 30 arranged to regulate a dispensing action of the material cartridge 2. The dispensing regulating unit 30 may *e.g.* regulate the volume, time and speed of fluid expelled from the nozzle 3 during a dispensing action. The material cartridge 2 and the dispensing regulating unit 30 may be fixed relative to each other upon movement of the material cartridge 2 and the nozzle temperature-regulating zone 5 relative to each other between the first position A and the second position B. The dispensing regulating unit 30 may be any in known in the art dispensing regulating unit. The dispensing regulating unit 30 may comprise a stepper motor actuated syringe pump consisting of a motor, syringe/compartment, and plunger arm. In one embodiment, the dispensing regulating unit 30 may be pneumatically action powered by a pump, compressor or a piston connected to a stepper motor. The dispensing regulating unit 30 may comprise connected or interacting parts 30a, 30b, 30c, 30d, 30e, 30f, 30g as illustrated in *e.g.* Fig. 3a. The dispensing regulating unit 30 may as illustrated in the figures comprise a part 30a, which is arranged in physical connection with the material cartridge 2. If the material cartridge comprises a piston 2b arranged to press material out of the body 2a of the material cartridge said part 30a of the dispensing regulating unit 30 is connected to the piston 2b and regulates the position of the piston 2b in the piston body 2a.

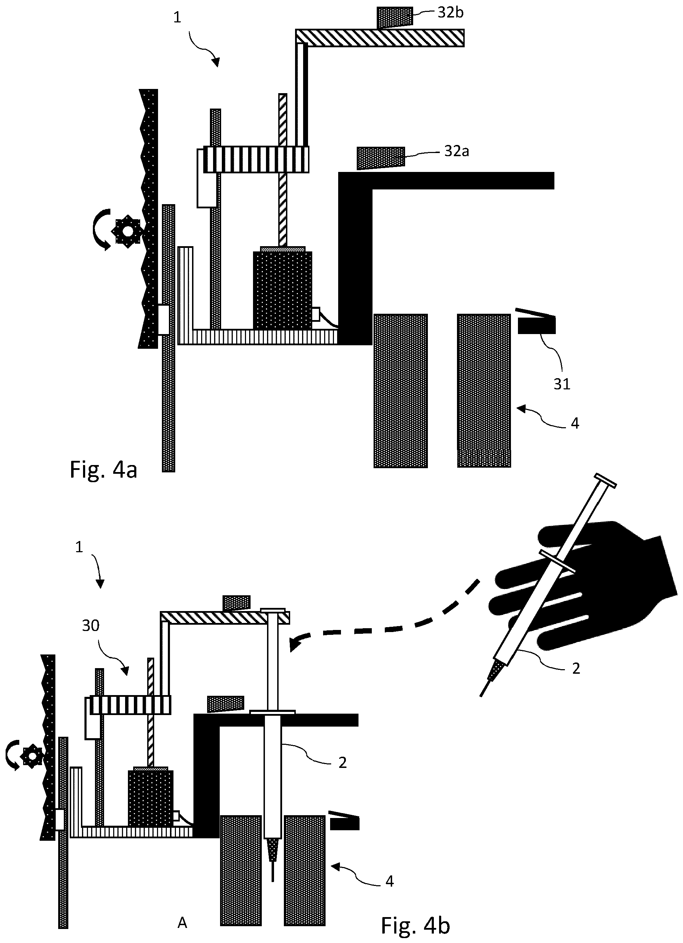

As illustrated in Figs 3a, 3b and 4a-4c, the positioning unit 6 may comprise a rack 6a and gear 6b, which upon interaction moves the material cartridge 2 and the nozzle temperature-regulating zone 5 relative to each other between the first position A and the second position B. The positioning unit 6 may comprise additional parts 6c, 6d, 6e in addition to the rack 6a and gear 6b.

A computer program comprising computer program code may, when executed, cause the positioning unit 6 to move the material cartridge 2 and nozzle temperature-regulating zone 5 relative to each other between the first position A and the second position B. The actuation of the positioning unit 6 may, hence, be an automatic process in accordance with a preprogramed dispensing program.

In Fig. 3a is illustrated the material cartridge 2 and the nozzle temperature-regulating zone 5 in the second position B relative to each other and the material cartridge 2 is in a dispensing position. In the embodiment shown the nozzle temperature-regulating zone 5 is stationary and the material cartridge 2 movable. When actuating the positioning unit 6 a portion of a fluid filled in the material cartridge 2 may be arranged to be supplied on a substrate through the nozzle 3. A switch 31 may be arranged such that when the material cartridge 2 and the nozzle-regulating zone 5 has reached a first position A relative to each other, the switch 31 is activated giving a signal to the positioning unit 6 such that this becomes activated and material dispensed through the nozzle 3.

In Fig. 3b is illustrated the material cartridge 2 and the nozzle temperature-regulating zone 5 in the first position A relative to each other and the material cartridge 2 is in a non-dispensing position. Here the switch 31 is not activated and no signal is given to the positioning unit 6 to dispense material through the nozzle 3.

In Fig. 4a is illustrated a dispensing system 1 with a removed material cartridge. In Fig. 4b is illustrated placement of a material cartridge 2, here a syringe, in the dispensing system 1. The material cartridge being placed in the dispensing system 1 such that the material cartridge 2 and the nozzle temperature-regulating zone 5 are in a first position A relative each other and the material cartridge 2 is in a non-dispensing position.

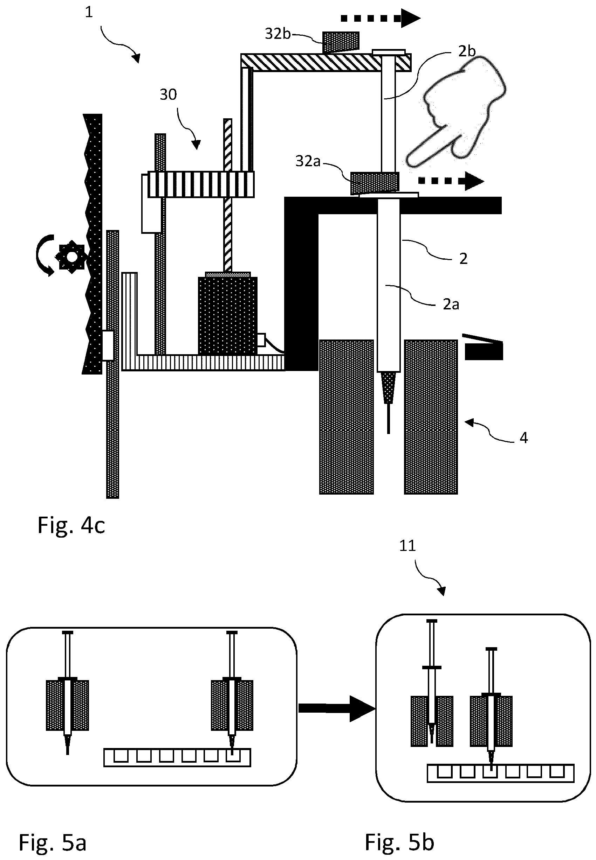

The material cartridge 2 may be locked into a correct position in the dispensing system 1 by means of at least one locking member 32a, 32b. In Fig. 4c the material cartridge being a syringe is locked into place by means of two locking members 32a, 32b. One locking member 32a locking the body 2a of the syringe to the positioning unit 6 and another locking member 32b locking the plunger 2b of the syringe to the dispensing regulating unit 30.

In Fig. 5b is illustrated a multi-dispensing system 11 comprising two or more of the dispensing systems 1 described above illustrating that the system 11 can be made smaller and more compact if the material cartridge nozzle 3 is moved into the temperature-regulating zone 5 when in a non-printing mode as compared to the multi-dispensing system shown in Fig. 5a where there is no movement of the material cartridge nozzle into the temperature-regulating zone when in a non-printing mode.

A 3D printer or 3D bioprinter may comprise one or more of the illustrated dispensing systems 1 and /or multi-dispensing systems 11. A biodispensing system may comprise one or more of the illustrated dispensing systems 1 and /or multi-dispensing systems 11.

## Claims

1. A dispensing system (1) comprising:
a material cartridge (2) provided with a nozzle (3),
a temperature-regulating unit (4) provided with a nozzle temperature-regulating zone (5) arranged to regulate the temperature of the material cartridge nozzle (3) when the material cartridge nozzle (3) is arranged within the nozzle temperature-regulating zone (5),
a positioning unit (6) arranged for movement of the material cartridge (2) and the nozzle temperature-regulating zone (5) relative to each other between a first position (A) and a second position (B),
wherein in the first position (A) the material cartridge nozzle (3) is arranged within the nozzle temperature-regulating zone (5),
wherein in the second position (B) at least a leading end portion (3a) of the material cartridge nozzle (3) is arranged outside the nozzle temperature-regulating zone (5).

2. The dispensing system (1) of claim 1, wherein the temperature-regulating unit (4) is arranged to regulate the temperature of the material cartridge nozzle (3) to a temperature within a range of -10°C to 20°C and/or 20 to 120°C.

3. The dispensing system (1) of any of the preceding claims, wherein the temperature-regulating unit (4) comprises a hollow compartment (7), at least a portion thereof forming the temperature regulating zone (5), the hollow compartment comprising a nozzle opening (8), wherein when the material cartridge (2) and the nozzle temperature-regulating zone (5) are in the first position (A) relative to each other the material cartridge nozzle (3) is arranged in the hollow compartment (7) in the temperature-regulating zone (5), and when the material cartridge (2) and the nozzle temperature-regulating zone (5) are in the second position (B) relative to each other at least the leading end portion (3a) of the material cartridge nozzle (3) extends out of the hollow compartment (7) through said nozzle opening (8).

4. The dispensing system (1) of claim 3 when dependent on claim 2, wherein the temperature-regulating unit (4) further comprises a) a Peltier element (20) arranged to transfer heat from the compartment (7) to a heat transferring element (21) connected to the Peltier element (20) and arranged to transfer heat generated by the Peltier element (20) and dissipate the transferred heat away from the Peltier element (20) or b) a Peltier element (20) arranged to heat the compartment (7).

5. The dispensing system (1) of any of the preceding claims, wherein when the material cartridge (2) and the nozzle temperature-regulating zone (5) are in the second position (B) relative to each other the material cartridge (2) is arranged in a dispensing position, and when the material cartridge (2) and the temperature-regulating zone (5) are in the first position (A) relative to each other the material cartridge (2) is arranged in a non-dispensing position.

6. The dispensing system (1) of any of the preceding claims, wherein the relative movement of the material cartridge (2) and the temperature-regulating zone (5) between the first (A) and second position (B) is a substantially vertical movement.

7. The dispensing system (1) of any of the preceding claims, wherein the material cartridge (2) is stationary and movement of the material cartridge (2) and the nozzle temperature-regulating zone (5) relative to each other between the first position (A) and the second position (B) is accomplished by movement of the nozzle temperature-regulating zone (5).

8. The dispensing system (1) of claim 7 when dependent on claim 3, wherein the nozzle temperature-regulating zone (5) is a moveable portion of a stationary hollow compartment (7).

9. The dispensing system (1) of any of claims 1-8, wherein the nozzle temperature-regulating zone (5) is stationary and movement of the material cartridge (2) and the nozzle temperature-regulating zone (5) relative to each other between the first position (A) and the second position (B) is accomplished by movement of the material cartridge (2).

10. The dispensing system (1) of any of claims 1-8, wherein movement of the material cartridge (2) and the nozzle temperature-regulating zone (5) relative to each other between the first position (A) and the second position (B) is accomplished by movement of both the material cartridge (2) and the nozzle temperature-regulating zone (5).

11. The dispensing system (1) of claim 9 or 10, wherein the material cartridge (2) comprises a body (2a) and a plunger (2b) arranged to be linearly pulled and pushed along the inside of the body, wherein upon movement of the material cartridge (2) the position of the plunger (2b) relative the body (2a) is kept constant.

12. The dispensing system (1) of any of the preceding claims further comprising a dispensing regulating unit (30) arranged to regulate a dispensing action of the material cartridge (2), optionally wherein the material cartridge (2) and the dispensing regulating unit (30) are fixed relative to each other upon movement of the material cartridge (2) and the nozzle temperature-regulating zone (5) relative to each other between the first position (A) and the second position (B).

13. A multi-dispensing system (11) comprising two or more of the dispensing systems (1) of any of claims 1-12.

14. A 3D bioprinter or biodispensing system comprising one or more of the dispensing systems of any of claims 1-12 and/or one or more multi-dispensing systems (11) of claim 13.

15. A method of regulating the temperature of a nozzle (3) of a material cartridge (2), the method comprising:
- providing a material cartridge (2) with a nozzle (3),
- providing a temperature-regulating unit (4) provided with a nozzle temperature-regulating zone (5),
- providing a positioning unit (6) for movement of the material cartridge (2) and the nozzle temperature-regulating zone (5) relative to each other between a first position (A) and a second position (B),
wherein in said first position (A) the temperature-regulating unit is arranged to regulate the temperature of the material cartridge nozzle (3), and in the second position (B) at least a leading end portion (3a) of the material cartridge nozzle (3) is arranged outside the nozzle temperature-regulating zone (5).
